# EUROPEAN PATENT SPECIFICATION

(11) **EP 1 966 587 B1**
(45) Date of publication and mention of the grant of the patent: **25.07.2018**
(21) Application number: 06847664.7
(22) Date of filing: 14.12.2006
(51) Int. Cl.: B01L 3/00, G01N 15/00, B01L 9/00, G01N 33/50, G01N 15/14

(54) **DIFFERENTIAL WHITE BLOOD COUNT ON A DISPOSABLE CARD**
DIFFERENTIELLE LEUKOZYTENZÄHLUNG AUF EINER EINWEGKARTE
NUMÉRATION DIFFÉRENTIELLE DE GLOBULES BLANCS SUR UNE CARTE JETABLE

(30) Priority: 30.12.2005 US 306508
(43) Date of publication of application: 10.09.2008
(73) Proprietor: Honeywell International Inc., Morris Plains, NJ 07950 (US)
(72) Inventor: FRITZ, Bernard, S., Eagan, MN 55123 (US); PADMANABHAN, Aravind, Plymouth, MN 55446 (US); REUTIMAN, Peter, Crystal, MN 55422 (US)
(74) Representative: Houghton, Mark Phillip
(86) International application number: PCT/US2006/047792
(87) International publication number: WO 2007/084232

(56) References cited:
- US-A1- 2005 105 077
- US-A1- 2005 255 600
- US-B1- 6 632 676

## Description

### Background

This present invention generally pertains to cytometry and particularly to portable cytometry. More particularly, the invention pertains to blood analysis.

US2005/255600 discloses a sample analyzer cartridge for use at a point of care of a patient such as in a doctor's office, in the home, or elsewhere in the field. US6632676 discloses a novel reagent system for use with automated and semi-automated hematology analyzers including an essentially isotonic blood diluting reagent, a blood cell lysing and hemoglobin conversion reagent, and a second lysing reagent for differentiating white blood cells into classes by size and functional characteristics.

### Summary

The present invention may include a fluid analysis card for achieving multipart differentiation of white blood cells, and is set out in accordance with the appended claims.

### Brief Description of the Drawing

Figure 1 is a perspective view of an illustrative sample analyzer and cartridge;
Figure 2 is a schematic view of the illustrative sample analyzer and cartridge of Figure 1;
Figure 3 is a more detailed schematic diagram showing the flow control of the sample analyzer and cartridge of Figure 2;
Figure 4a is a diagram of an illustrative cartridge having various analysis circuits;
Figure 4b shows a planar view of the illustrative cartridge incorporating the circuits of Figure 4a;
Figure 5 is a schematic view of a number of illustrative storage reservoirs that can be included in a cartridge;
Figure 6 is a schematic flow diagram showing an illustrative method for analyzing a blood sample;
Figure 7 is a schematic flow diagram showing another illustrative method for analyzing a blood sample;
Figure 8 is a schematic diagram of various components for a multiple part measurement approach;
Figure 9a is a diagram of an illustrative cartridge having various analysis circuits;
Figure 9b shows a planar view of the illustrative cartridge incorporating the circuits of Figure 9a;
Figure 9c shows a planar view of the illustrative cartridge incorporating the circuits of Figure 9a;
Figure 10a is a diagram of another illustrative cartridge having various analysis circuits;
Figure 10b shows a planar view of the illustrative cartridge incorporating the circuits of Figure 10a;
Figure 10c shows a planar view of the illustrative cartridge incorporating the circuits of Figure 10a;
Figure 11a is a diagram of another illustrative cartridge having various analysis circuits;
Figure 11b shows a planar view of the illustrative cartridge incorporating the circuits of Figure 11a;
Figure 12a is a diagram of another illustrative cartridge having various analysis circuits;
Figure 12b shows a planar view of the illustrative cartridge incorporating the circuits of Figure 12a;
Figure 12c shows a planar view of the illustrative cartridge incorporating the circuits of Figure 12a;
Figure 13a is a diagram of another illustrative cartridge having various analysis circuits;
Figure 13b shows a planar view of the illustrative cartridge incorporating the circuits of Figure 13a;
Figure 13c shows a planar view of the illustrative cartridge incorporating the circuits of Figure 13a;
Figure 14a is a diagram of another illustrative cartridge having various analysis circuits;
Figure 14b shows a planar view of the illustrative cartridge incorporating the circuits of Figure 14a; and
Figures 15a and 15b reveal data and plots of four-part differentiation of blood cells.

### Description

The present invention generally relates to sample analyzers, and more particular, to sample analyzers with removable and/or disposable cartridges for use at the point of care of a patient such as in a doctor's office, in the home, or elsewhere in the field. By providing a removable and/or disposable cartridge with all of the needed reagents and/or fluids, the sample analyzer can be reliably used outside of the laboratory environment, with little or no specialized training. This may, for example, help streamline the sample analysis process, reduce the cost and burden on medical or other personnel, and increase the convenience of sample analysis for many patients, including those that require relatively frequent blood monitoring/analysis.

A method which allows rapid and efficient particle discrimination in a particle-suspension sample is flow cytometry. In this method, a suspension of particles, typically cells in a blood sample, is transported through a flow channel where the individual particles in the sample are illuminated with one or more focused light beams. The interaction of the light beam(s) with the individual particles flowing through the flow channel is detected by one or more light detectors. Commonly, the detectors are designed to measure light absorption or fluorescence emission, at specific beam or emission wavelengths, and/or light scattering at specific scattering angles. Thus, each particle that passes through the flow channel can be characterized as to one or more features related to its absorption, fluorescence, light scattering or other optical or electrical properties. The properties that are measured by the detectors may allow each particle to be mapped into a feature space whose axes are the light intensities or other properties which are measured by the detectors. In the ideal, the different particles in the sample map into distinct and non-overlapping regions of the feature space, allowing each particle to be analyzed based on its mapping in the feature space. Such analysis may include counting, identifying, quantifying (as to one or more physical characteristics) and/or sorting of the particles.

In one illustrative example may be a sample analyzer which is provided that has a removable cartridge that receives a collected sample, such as a collected whole blood sample, and once the removable cartridge is installed and the analyzer is activated, the analyzer and cartridge automatically processes the sample and the analyzer provides sufficient information for the user to make a clinical decision. In some examples, the analyzer displays or prints out quantitative results (e.g., inside and/or outside of a predefined range), such that no further calculations or interpretation is required by the user.

The sample analyzer may be used to, for example, determine the number and/or types of white blood cells in a blood sample. In one illustrative example, the analyzer includes a housing and a removable fluidic cartridge, wherein the housing is adapted to receive the removable fluidic cartridge. In some cases, the removable fluidic cartridge is a disposable cartridge. In one illustrative example, the removable fluidic cartridge may include one or more reagents (e.g., lysing reagents, stain, dilutent, and so on), one or more analysis channels, one or more flow sensors, one or more valves, and/or a fluidic circuit that is adapted to process (e.g., lyse, stain, mix, and so forth) a sample and deliver processed sample(s) to the appropriate analysis channel on the cartridge. To support the card, the housing may include, for example, a pressure source, one or more light sources, one or more light detectors, a processor and a power source. The pressure source may provide appropriate pressure(s) to the removable fluidic cartridge ports to drive the fluids as required through the fluidic circuit. The one or more light sources of the analyzer may be used to interrogate the prepared sample in at least selected analysis channels of the removable cartridge, and the one or more light detectors of the analyzer may detect the light that passes through, is absorbed by and/or is scattered by the sample. The processor may be coupled to at least some of the light sources and detectors, and may determine one or more parameters of the sample. In some examples, the one or more analysis channels on the removable fluidic cartridge may include one or more flow cytometry channels.

In some illustrative examples, a whole blood sample may be provided to the removable fluidic cartridge, and the removable cartridge may be adapted to perform a blood analysis.

To count and classify white blood cells, at least a portion of the whole blood sample may be provided to a white blood measurement channel in the removable cartridge. The blood sample provided to the white blood measurement channel may be, for example, diluted if desired, the red blood cells may be lysed on the fly, the resulting sample may be hydrodynamically focused for core formation and ultimately provided to a second cytometry channel. The cytometry channel may also be located along or under a transparent flow stream window of the removable cartridge so that the cells in the flow stream can be optically interrogated by a corresponding light source and detector. In some cases, a flow sensor may be provided on the removable cartridge to provide a measure of the flow rate through the second cytometry channel.

In some cases, illustrative measured parameters of the white blood cell measurement channel may include, for example, two (2), three (3), four (4) or five (5) part white blood cell differentiation, total white blood cell count and/or on-axis white blood cell volume. For white blood cell differentiation, the first, second, third, fourth and fifth parts, types or kinds of white blood cells, may refer to lymphocytes, monocytes, neutrophils, eosinophils and basophils, respectively. The white blood cells may also be classified by some into three groups which include lymphocytes, monocytes and granulocytes (LMG). The granulocytes may include neutrophils, eosinophils and basophils.

Five other parameters may also be measured or calculated, depending on the desired application. In some cases, stains and/or fluorescent tags may be added to the sample prior to providing the sample to the cytometry channel, which in some cases, may aid in cell differentiation.

Several general types of light scattering measurements may be made in flow cytometry. Light intensity measurements made at small angles (about 1 - 25 degrees with respect to the incident light beam), usually, called forward or small-angle scattering, give information on cell size. Forward scattering also strongly depends on the difference of refraction between cells and the extra-cellular medium, so that cells with damaged membranes, for example, can be distinguished. Light intensity measurements made at an angle of about 65 degrees -115 degrees from the incident light, usually referred to as orthogonal or large angle scattering, can provide information about the size and degree of structure of particles.

Simultaneous light scattering measurements at different angles or in combination with absorption or fluorescence measurements may be used in flow cytometry methods. For example, absorption of light in combination with light scattering can be used in flow cytometry to distinguish between various kinds of white cells. This method sometimes uses a staining of the cells.

Typically, such particle discrimination methods are implemented, at least in part, using one or more pieces of equipment, collectively herein called a sample analyzer. Many sample analyzers are rather large devices that are used in a laboratory environment by trained personnel. To use many sample analyzers, a collected sample must first be processed, such as by diluting the sample to a desired level, adding appropriate reagents, centrifuging the sample to provide a desired separation, and the like, prior to providing the prepared sample to the sample analyzer. To achieve an accurate result, such sample processing must typically be performed by trained personnel, which can increase the cost and time required to perform the sample analysis.

Many sample analyzers also require operator intervention during the analysis phase, such as requiring additional information input or additional processing of the sample. This can further increase the cost and time required to perform a desired sample analysis. Also, many sample analyzers merely provide raw analysis data as an output, and further calculations and/or interpretation must often be performed by trained personnel to make an appropriate clinical decision.

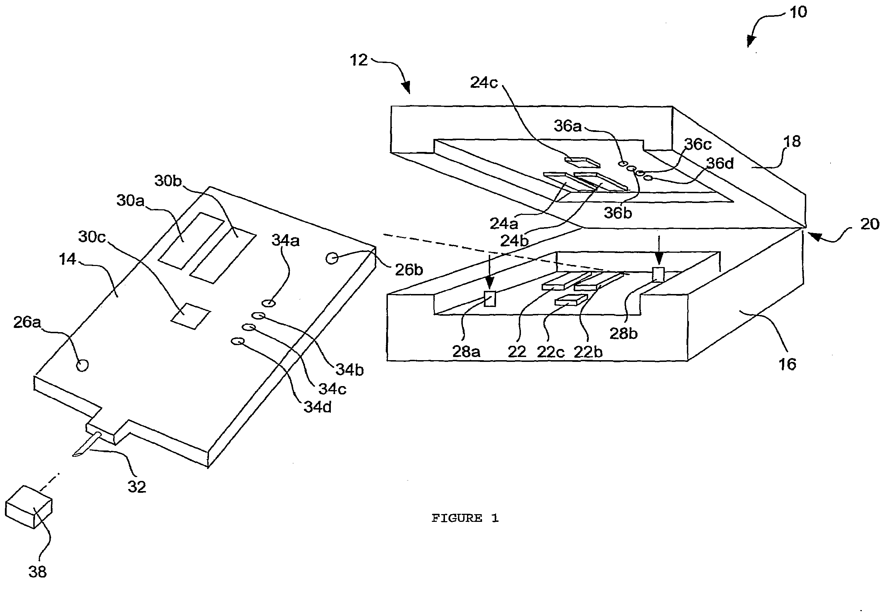

Figure 1 is a perspective view of an illustrative sample analyzer and cartridge. The illustrative sample analyzer is generally shown at 10, and includes a housing 12 and a removable or disposable cartridge 14. The illustrative housing 12 includes a base 16, a cover 18, and a hinge 20 that attaches the base 16 to the cover 18, but this is not required. In the illustrative example, the base 16 includes a first light source 22a, a second light source 22b, and a third light source 22c, along with associated optics and the necessary electronics for operation of the sample analyzer. Each of the light sources may be a single light source or multiple light sources, depending on the application. In some cases, the overall dimensions of the housing may be less than 1 cubic foot, less than one-half cubic foot, less than one-quarter cubic foot, or smaller, as desired. Likewise, the overall weight of the housing may be less than 10 pounds, less than 5 pounds, less than one pound, or less, as desired.

The illustrative cover 12 includes a pressure source (e.g. pressure-chambers with control microvalves), a first light detector 24a, a second light detector 22b, and a third light detector 22c, each with associated optics and electronics. Each of the light detectors may also be a single light detector or multiple light detectors, depending on the application. Polarizers and/or filters may also be provided, if desired, depending on the application.

The illustrative removable cartridge 14 is adapted to receive a sample fluid via a sample collector port, which in the illustrative example, includes a lancet 32. The lancet 32 may be retractable and/or spring loaded, in some examples. A cap 38 may be used to protect the sample collector port and/or lancet 32 when the removable cartridge 14 is not in use.

In the illustrative example, the removable cartridge 14 performs a blood analysis on a whole blood sample. The lancet 32 may be used to prick the finger of the user to produce a sample of blood, which through capillary action, may be drawn into an anti-coagulant coated capillary in the removable cartridge 14. The removable cartridge 14 may be constructed similar to the fluidic circuits available from Micronics Technologies, some of which are fabricated using a laminated structure with etched channels. However, it is contemplated that the removable cartridge 14 may be constructed in any suitable manner including by injection molding or any other suitable manufacturing process or method, as desired.

During use, and after a blood sample has been drawn into the removable cartridge 14, the removable cartridge 14 may be inserted into the housing when the cover 18 is in the open position. In some cases, the removable cartridge 14 may include holes 26a and 26b for receiving registration pins 28a and 28b in the base 16, which may help provide alignment and coupling between the different parts of the instrument. The removable cartridge 14 may also include a first transparent flow stream window 30a, a second transparent flow stream window 30b and a third transparent window 30c, which are in alignment with the first, second and third light sources 22a, 22b and 22c, and the first, second and third light detectors 24a, 24b and 24c, respectively.

When the cover is moved to the closed position, and the system is pressurized, the cover 18 may provide controlled pressures via pressure providing ports 36a, 36b, 36c, and 36d to pressure receiving ports 34a, 34b, 34c and 34d, respectively, in the illustrative removable cartridge 14. It is contemplated that more or less pressure providing and pressure receiving ports may be used, depending on the application. Alternatively, or in addition, it is contemplated that one or more micro-pumps, such as electrostatically actuated meso pumps, may be provided on or in the removable cartridge 14 to provide the necessary pressures to operate the fluidic circuit on the removable cartridge 14. Some illustrative electrostatically actuated meso pumps are described in, for example, U.S. Patent Numbers 5,836,750, 6,106,245, 6179,586, 6,729,856, and 6,767,190, all assigned to the assignee of the present invention.

Once pressurized, the illustrative instrument may perform a blood analysis on the collected blood sample. In some cases, the blood analysis may include a white blood cell count (WBC).

To count and classify white blood cells, the whole blood sample may be provided to a white blood measurement channel in the removable cartridge 14. The blood sample may then be diluted if desired, the red blood cells may be lysed on the fly, the resulting sample may be hydrodynamically focused for core formation and ultimately provided to a second cytometry channel. The cytometry channel may be located along the second transparent flow stream window 30b of the removable cartridge 14 so that the cells in the flow stream can be optically interrogated by the second light source 22b and the second light detector 24b. A flow sensor may be provided on the removable cartridge 14 to provide a measure of the flow rate through the cytometry channel. In some cases, measured white blood cell parameters may include, for example, three (3) or (5) part white cell differentiation, total white blood cell count and/or on-axis white blood cell volume. Other parameters may also be measured or calculated, depending on the application.

Even though Figure 1 shows one illustrative sample analyzer and cartridge assembly, it is contemplated that other sample analyzer configurations may be used. For example, the sample analyzer 10 and removable cartridge may be similar to that described in U.S. Patent Application 2004/0211077 to Schwichtenberg et al..

In some cases, the sample analyzer 10 may be adapted to be used at the point of care of a patient such as in a doctor's office, in the home, or elsewhere in the field. The ability to provide a sample analyzer 10 that can be reliably used outside of the laboratory environment, with little or no specialized training, may help streamline the sample analysis process, reduce the cost and burden on medical personnel, and increase the convenience of sample analysis for many patients, including those that require relatively frequent blood monitoring/analysis.

During operation, the sample analyzer 10 may receive a collected sample, such as a collected whole blood sample, and once the analyzer is activated, the sample analyzer 10 may automatically process the sample and provide information to the user to make a clinical decision. In some examples, the sample analyzer 10 may display or print out quantitative results (e.g., inside and/or outside of a predefined range), such that no further calculations or interpretation is required by the user.

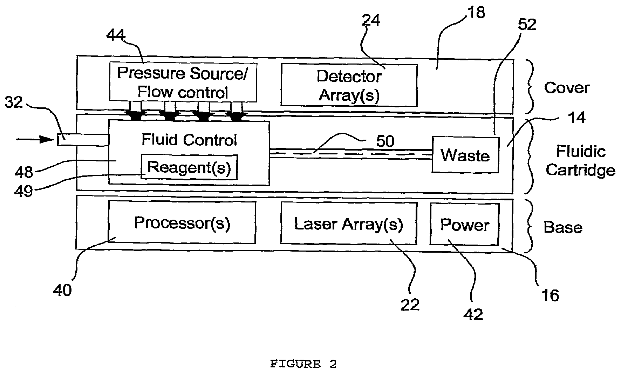

Figure 2 is a schematic view of the illustrative sample analyzer and cartridge of Figure 1. As detailed above, and in the illustrative example, the base 16 may include a number of light sources 22, associated optics and the necessary control and processing electronics 40 for operation of the analyzer. The base 16 may also include a battery 42, transformer or other power source. The cover 12 is shown having a pressure source/flow control block 44 and a number of light detectors 24 with associated optics.

The removable cartridge 14 may receive a sample fluid via the sample collector port or lancet 32. When pressurized by the pressure source/flow control block 44, the removable cartridge 14 may perform a blood analysis on the received blood sample. In some examples, and as described above, the removable cartridge 14 may include a number or reagents 49, and a fluidic circuit for mixing the reagents with the blood sample to prepare the blood sample for analysis. Also, the removable cartridge 14 may include a number of flow sensors to help control and/or verify the proper operation of the fluidic circuit.

In some cases, the blood sample is prepared (e.g., lysed, stained, diluted and/or otherwise processed) and then hydrodynamically focused for core formation in one or more on-board cytometry channels, such as cytometry channel 50. In the illustrative example, the cytometry channel 50 may be routed past a transparent flow stream window such as the first transparent flow stream window 30a in the removable cartridge 14. An array of light sources 22 and associated optics in the base 16 may provide light through the core stream via the flow stream window 30a. An array of light detectors 24 and associated optics may receive scattered and non-scattered light from the core, also via the flow stream window 30a. The controller or processor 40 may receive output signals from the array of detectors 24, and may differentiate and/or counts selected cells that are present in the core stream.

It is contemplated that the removable cartridge 14 may include a fluid control block 48 for helping to control the velocity of at least some of the fluids on the removable cartridge 14. In the illustrative example, the fluid control block 48 may include flow sensors for sensing the velocity of the various fluids and report the velocities to the controller or processor 40. The controller or processor 40 may then adjust one or more control signals, which are provided to the pressure source/flow control block 44, to achieve the desired pressures and thus the desired fluid velocities for proper operation of the analyzer.

Because blood and other biological waste can spread disease, the removable cartridge 14 may include a waste reservoir 52 downstream of the illustrative cytometry channel 50. The waste reservoir 52 may receive and store the fluid of the flow stream in the removable cartridge 14. When a test is completed, the removable cartridge 14 may be removed from the analyzer and disposed of, preferably in a container compatible with biological waste.

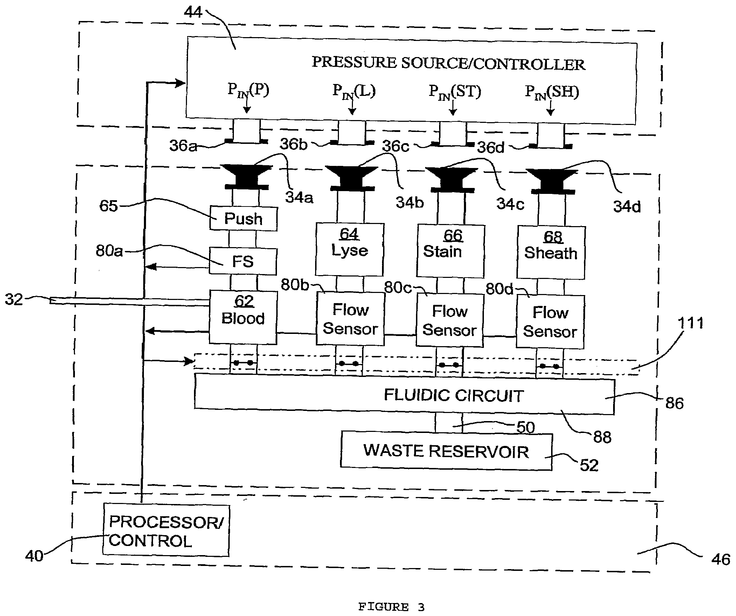

Figure 3 is a more detailed schematic diagram showing the flow control of the sample analyzer and cartridge of Figure 2. In the illustrative example, the pressure source/flow controller 44 in the cover 18 provides five controlled pressures including a sample push (P) pressure 36a, a lyse (L) pressure 36b, a stain (ST) pressure 36c, and a sheath (SH) pressure 36d. These are only illustrative, and it is contemplated that more, less or different pressures (e.g., diluent pressure to a diluent reservoir) may be provided by pressure source/flow controller 44, depending on the application. Also, it is contemplated that the cover 18 may not include a pressure source/flow controller 44. Instead, the removable cartridge 14 may include an on-board pressure source, such as a compressed air reservoir, one or more micro-pumps such as electrostatically actuated meso pumps as described above, or any other suitable pressure source, as desired. The array of light sources and detectors are not shown in Figure 3.

In the illustrative example, pressure source 36a provides pressure to a blood sample reservoir 62 via a pusher fluid 65, pressure source 36b provides pressure to a lyse reservoir 64, pressure source 36c provides pressure to a stain reservoir 66, and pressure source 36d provides pressure to a sheath reservoir 68.

In one illustrative example, each pressure source may include a first pressure chamber for receiving an input pressure, and a second pressure chamber for providing a controlled pressure to the removable cartridge. A first valve may be provided between the first pressure chamber and the second pressure chamber for controllably releasing the pressure in the first pressure chamber to the second pressure chamber. A second valve, in fluid communication with the second pressure chamber, may controllably vent the pressure in the second pressure chamber to atmosphere. This may allow the pressure source/flow controller 44 to provide a controlled pressure to each of the pressure receiving ports on the removable cartridge 14. Each valve may be an array of electrostatically actuated microvalves that are individually addressable and controllable, as described in, for example, co-pending U.S. Patent Application Serial Number 09/404,560, entitled "Addressable Valve Arrays for Proportional Pressure or Flow Control". Alternatively, each valve may be an array of electrostatically actuated microvalves that are pulse modulated with a controllable duty cycle to achieve a controlled "effective" flow or leak rate. Other valves may also be used, if desired.

The illustrative removable cartridge 14 includes five pressure receiving ports 34a, 34b, 34c and 34d, each for receiving a corresponding controlled pressure from the pressure source/flow controller 44. In the illustrative example, the pressure receiving ports 34a, 34b, 34c, and 34d may direct the controlled pressures to the blood reservoir 62, the lyse reservoir 64, the stain reservoir 66, and the sheath reservoir 68, respectively. The lyse reservoir 64, stain reservoir 66 and sheath reservoir 68 may be filled before the removable cartridge 14 is shipped for use, while the blood reservoir 62 may be filled in the field via sample collector port or lancet 32.

As shown, a flow sensor may be provided in-line with each or selected fluids. Each flow sensor 80a, SOb, 80c and SOd may measure the velocity of the corresponding fluid. The flow sensors 80a, 80b, 80c and 80d are preferably thermal anemometer type flow sensors, and more preferably microbridge type flow sensor. Microbridge flow sensors are described in, for example, U.S. Patent No. 4,478,076, U.S. Patent No. 4,478,077, U.S. Patent No. 4,501,144, U.S. Patent No. 4,651,564, U.S. Patent No. 4,683,159, and
U.S. Patent No. 5,050429. An output signal from each flow sensor 80a-80d may be provided to controller or processor 40. The controller or processor 40 may provide control signals to the pressure source/controller 44, as shown. For example, to control the pressure provided to the blood sample, the controller or processor 40 may open a first valve between a first pressure chamber and a second pressure chamber in the pressure source/controller 44 for controllably releasing a pressure in the first pressure chamber to the second pressure chamber when the velocity of the blood sample drops below a first predetermined value. Likewise, the controller or processor 40 may open a second valve that vent the pressure in the second pressure chamber when the velocity of the blood sample increases above a second predetermined value. The controller or processor 40 may control the velocities of the lysing reagent, stain, and sheath fluid in a similar manner.

In some cases, the controller or processor 40 may detect one or more changes in the flow rate passing through a flow channel. A change in flow rate may correspond to, for example, one or more bubbles in a flow channel, an occlusion or partial occlusion of a flow channel caused by. for example, coagulation of the blood sample, unwanted or foreign objects in a flow channel, and/or other undesirable characteristics of a flow channel. The controller or processor 40 may be programmed to detect such characteristics from the flow rate, and in some cases, issue a warning and/or shut down the sample analyzer.

Thermal anemometer type flow sensors typically include a heater element that, when energized, produces one or more heat pulses in the fluid, and further includes one or more heat sensors positioned upstream and/or downstream of the heater element to detect the one or more heat pulses. The velocity of the fluid through the flow channel may be related to the time that it takes for a heat pulse to travel from the heater element to one of the spaced heat sensors.

In some cases, thermal anemometer type flow sensors may be used to detect the thermal conductivity and/or specific heat of the fluid. Changes in the thermal conductivity and/or specific heat of the fluid may correspond to changes in the fluid characteristics, such as a change of state of the fluid (coagulation of a blood sample), bubbles in the fluid, unwanted or foreign objects in the fluid, etc. Thus, and in some examples, it is contemplated that the controller or processor 40 may detect characteristics of the fluid by monitoring the thermal conductivity and/or specific heat of the fluid that passes by the thermal anemometer type flow sensors.

In some cases, an impedance sensor may be provided in fluid communication with a flow channel. The controller or processor 40 may be coupled to the impedance sensor. Changes in the impedance of the fluid may indicate a change in fluid characteristics, such as a change in the state of the fluid (coagulation of a blood sample), bubbles in the fluid, unwanted or foreign objects in the fluid, etc. Thus, and in some examples, it is contemplated that the controller or processor 40 may detect characteristics of the fluid by monitoring the impedance of the fluid that passes by the impedance sensor.

Downstream valves generally shown at 111 may also be provided. Controller or processor 40 may open/close downstream valves 111, as desired. For example, the downstream valves 111 may remain closed until the system is fully pressurized. This may help prevent the blood, lysing reagent, sphering reagent, sheath fluid and diluent from flowing into the fluidic circuit 86 before the system is fully pressurized. Also, the downstream valves 111 may be controlled to aid in performing certain tests, like zero-flow tests, etc. In another example, downstream valves 111 may be opened by mechanical action when, for example, the cover is closed.

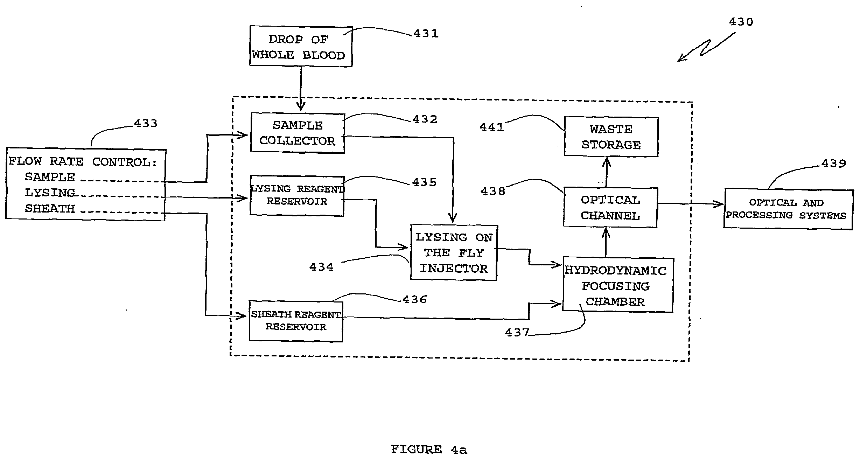

Figure 4a is a diagram or schematic of various components of a removable card or cartridge for a configuration 430 which may provide a four part differentiation of white blood cells with one run. A drop of whole blood may be provided from source 431 to a sample collector 432. The blood may be provided to a lysing on the fly injector mechanism or block 434. The flow rate of the blood to the block 434 may be controlled by a flow rate control mechanism or block 433. A lysing reagent may be provided from a lysing reagent reservoir 435 to the lysing on the fly injector 434 with a flow rate controlled by the flow rate control mechanism 433. Lysed blood may be provided from the lysing on the fly mechanism 434 to a hydrodynamic focusing chamber 437. A sheath reagent from a reservoir 436 may go to the focusing chamber 437 to provide a sheath around the blood cells as they enter an optical channel 438. The flow of the sheath reagent may be controlled by the control block 433. The blood cells may be differentiated by optical and processing systems 439. Scattering and analysis by the systems 439 may provide a four part differentiation of the cells. The cells and the associated fluids may go from the optical channel to a waste storage 441. The lysing and sheath reagents may be the same or different reagents. The sources of the lysing and sheath reagents may be reservoirs 435 and 436, respectively, on or off the card or cartridge. The waste storage 441 may be on or off the card or cartridge. The flow rate control mechanism 433 may be on or off the card or it may be partially on the card or cartridge.

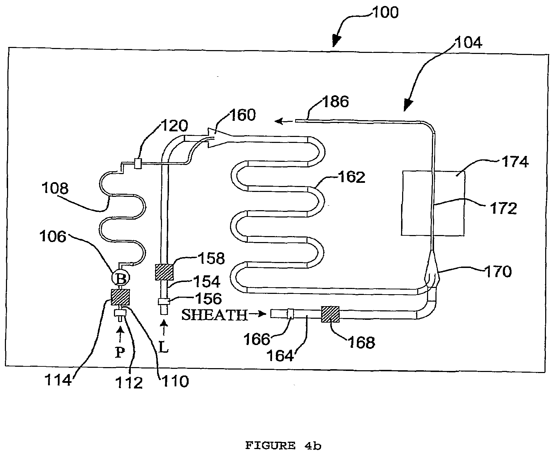

Figure 4b shows an implementation of the schematic of the configuration 430 of Figure 4a in an illustrative removable card or cartridge, which may be designed to be disposable. The cartridge is generally shown at 100 of this Figure, and may be similar to removable cartridge 14 shown and described above with reference to Figures 1, 2 and 3. It should be understood that the removable cartridge 100 is only illustrative, and that the present invention can be applied to many microfluidic cartridges, regardless of form, function or configuration. For example, the present invention may be applied to removable cartridges adapted for flow cytometry, hematology, clinical chemistry, blood chemistry analysis, urinalysis, blood gas analysis, virus analysis, bacteria analysis, electrolyte measurements, and so on. It is also contemplated that the removable cartridges of the present invention, such as removable cartridge 100, may be made from any suitable material or material system including, for example, glass, plastic, silicon, one or more polymers, hybrid material, or any other suitable material or material system, or combination of materials or material systems.

The illustrative removable cartridge 100 includes a measurement channel 104, although more or less measurement channels may be used, as desired. The measurement channel 104 is a white blood cell measurement channel. A whole blood sample is received by the removable cartridge 100 via blood receiving port 106, which through capillary action, draws in a known amount of blood into an anti-coagulant coated blood sample storage capillary 108. A sample push (P) pressure, such as a sample push (P) pressure 36a of Figure 3, is provided to a sample push fluid reservoir, such as sample push fluid reservoir 65 of Figure 3. When pressure is applied, the sample push fluid is forced from the sample push fluid reservoir into a blood sample push channel 110.

In some illustrative examples, a valve 112 and a flow sensor 114 may be provided in line with the blood sample push channel 110. The valve 112 may be controlled to open when it is desirable to push the blood sample through the fluidic circuit. The flow sensor 114 may measure the flow rate of the blood sample push fluid, and thus the blood sample flow rate through the anti-coagulant coated capillary 108. The flow rate provided by the flow sensor 114 may be used to help control the sample push (P) pressure that is provided to the removable cartridge 100.

In the illustrative example, the whole blood sample is provided the white blood cell measurement channel 104 via capillary 108. A valve 120 is provided to control the blood sample flow from capillary 108 into the white blood cell measurement channel 104.

As to the white blood cell measurement channel 104, a white blood cell lysing reagent (L) pressure, such as a lysing pressure (P_{IN}(L)) 36b of Figure 3, is provided to a lysing reagent reservoir, such as lyse reservoir 64 of Figure 3. When pressure is applied, the lysing reagent in the lyse reservoir 64 is forced into a lysing reagent tube or channel 154. A channel may in certain contexts mean a tube, a capillary, a serpentine flow path, a conveyance, or the like; however, the term "channel" may be used herein in a general sense.

In some illustrative examples, a valve 156 and a flow sensor 158 may be provided in line with the lysing reagent channel 154. The valve 156 may be controlled to open when it is desirable to push the lysing reagent into the fluidic circuit. The flow sensor 158 may measure the flow rate of the lysing reagent, and provide a measure of the lysing reagent flow rate through the lysing reagent channel 154. The flow rate provided by the flow sensor 158 may be used to help control the lysing pressure that is provided to the removable or disposable cartridge 100 by the pressure source/controller 44.

During normal functional operation of the illustrative removable cartridge 100, the lysing reagent is provided to a combining, coupling or intersecting region 160 at a lysing reagent flow rate, and the blood sample is provided to the intersecting region 160 at a blood sample flow rate. The blood sample flow rate and the lysing reagent flow rate may be controlled by a pressure source/controller, such as pressure source/controller 44 of Figure 3.

The intersecting region 160 may be configured so that the lysing reagent flows circumferentially around the blood sample when both fluids are flowing through the intersecting region 160. Even though the junction or combining, coupling or intersecting region 160 or 170 may be called by a term more descriptive of its purpose (e.g., hydrodynamic focusing chamber), the term "intersecting region" may be used in a general sense herein. In some cases, the lysing reagent flow rate may be higher than the blood sample flow rate, which may help improve the flow characteristics in a lysing-on-the-fly channel 162 (which may have a serpentine path), and in some cases, to help form a thin ribbon of blood that is completely and uniformly surrounded by the lysing reagent. Such a ribbon flow may help the lysing reagent uniformly lyse the red blood cells as they travel through the lysing-on-the-fly channel 162. Furthermore, the length of the lysing-on-the-fly channel 162, in conjunction with the flow rate of the lysing reagent and blood sample, may be set such that the blood sample is exposed to the lysing reagent for an appropriate amount of time.

A sheath fluid (SH) pressure, such as a sheath (SH) pressure 36d of Figure 3, may be provided to a sheath fluid reservoir, such as sheath fluid reservoir 68 of Figure 3. When pressure is applied, the sheath fluid is forced from the sheath fluid reservoir 68 into a sheath channel 164. In some illustrative examples, a valve 166 and a flow sensor 168 may be provided in line with a sheath channel 164. The valve 166 may be controlled to open when it is desirable to push the sheath fluid into the fluidic circuit. The flow sensor 168 may measure the flow rate of the sheath fluid, and may provide a measure of the sheath flow rate through the sheath channel 164. The flow rate provided by the flow sensor 168 may be used to help control the sheath pressure (SH) that is provided to the removable cartridge 100.

In the illustrative example, the sheath fluid is provided to an intersecting region 170 at a sheath fluid flow rate, and the lysed blood sample is provided to the intersecting region 170 at a lysed blood sample flow rate. Although this region 170 may be referred to as a sheath forming, hydrodynamically focusing, or other purpose effecting, or the like region, it will be generally referred to an "intersecting region" herein. The lysed blood sample flow rate and the sheath flow rate may be controlled by a pressure source/controller, such as pressure source/controller 44 of Figure 3.

The intersecting region 170 may be configured so that the sheath fluid flows circumferentially around the lysed blood sample when both fluids are flowing through the intersecting region 170. In some cases, the sheath flow rate is significantly higher than the lysed blood sample flow rate, which may help improve core formation in a downstream flow optical cytometry channel 172. For example, in some flow cytometry applications, the intersecting region 170 may be configured to hydrodynamically focus and arrange the white blood cells in the lysed blood sample in a single file core so that each white blood cell can be individually optically interrogated by an analyzer as they pass through an optical window region 174 in the removable cartridge 100. In some cases, the fluid that passes through the cytometry channel 172 is provided via a line or channel 186 to an on-board waste reservoir 52 of Figure 3.

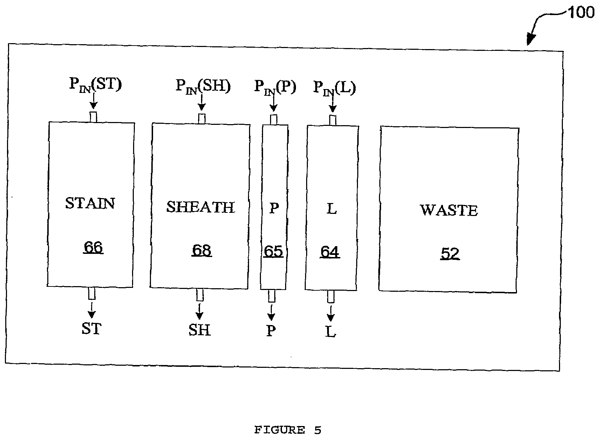

Figure 5 is a schematic view of a number of illustrative storage reservoirs that can be included in a removable cartridge. In the illustrative example, a removable cartridge such as removable cartridge 100 of Figure 4 may include, for example, a lysing reagent reservoir 64, a pusher fluid reservoir 65, a stain reservoir 66, a sheath fluid reservoir 68, and a waste reservoir 52. These are only illustrative, and it is contemplated that more, less or different reservoirs may be provided on or in a removable cartridge.

Each reservoir may be sized and include an appropriate amount of fluid and/or reagent to support the desired operation of the removable cartridge. Likewise, and in some examples, a reservoir such as stain reservoir 66 may be desirable to add a stain to the white blood cell channel to support white blood cell differentiation. It is contemplated that the reagents and/or fluids stored in the reservoirs may initially be in liquid or lyophilized form, depending on the application.

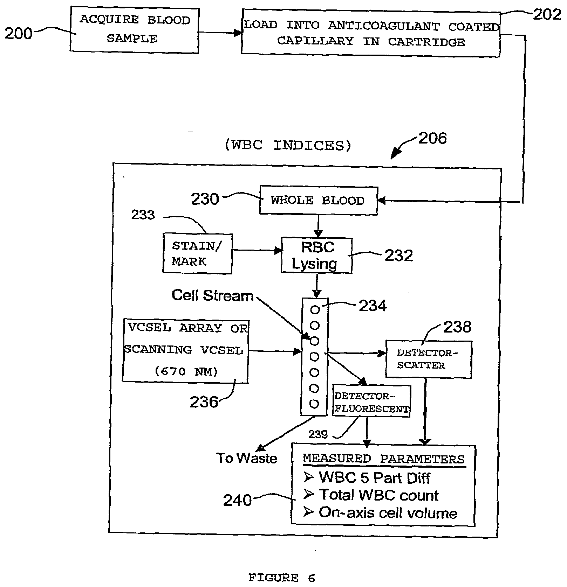

Figure 6 is a schematic flow diagram showing an illustrative method for analyzing a blood sample using a removable cartridge. In the illustrative method, a blood sample is first acquired at step 200. Next, the blood sample may be provided to an anti-coagulant coated capillary 202 in a removable cartridge. The blood sample may then be provided to a white blood cell (WBC) measurement channel 206.

In the illustrative WBC measurement channel 206, the red blood cells of the whole blood 230 may be first lysed as shown at 232, stained or marked as shown at 233, and then hydrodynamically focused and provided single file down a WBC cytometry channel 234 in the removable cartridge. A light source 236, such as a vertical cavity surface emitting laser (VCSEL), may shine light on the individual cells as they pass by an analysis region of the WBC cytometry channel 234. In some cases, an array of VCSEL devices may be provided, and only the VCSEL(s) that is/are aligned with the individual cells as they pass by the analysis region of the WBC cytometry channel 234 is activated. Some of the incident light provided by a VCSEL is scattered, and a detector 238 detects the scattered light. Other kinds of light sources may be used. In some cases, the detector 238 may detect forward angle scatter light (FALS), small angle scatter light (SALS), and large angle scatter light (LASL). A detector 239 may detect fluorescent light from some of the cells. In some cases, and as shown at 240, a number of parameters may be measured during the analysis including, for example, on-axis cell volume, total WBC count, and WBC five (5) part differentiation.

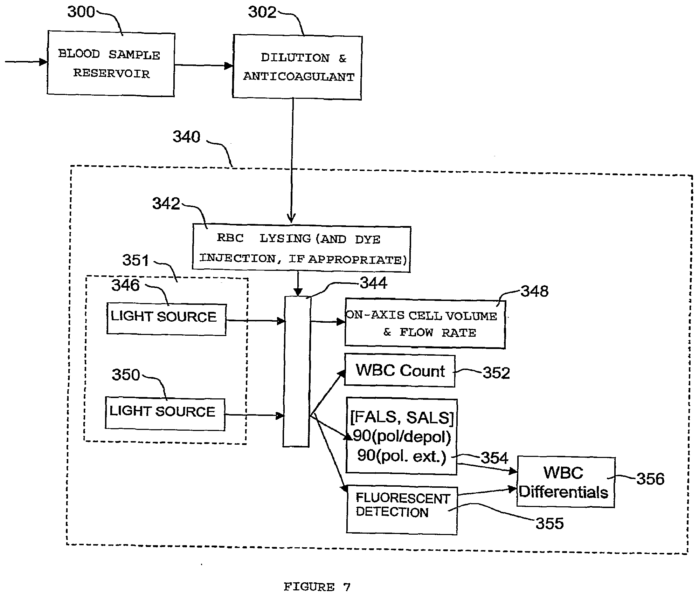

Figure 7 is a schematic flow diagram showing another illustrative method for analyzing a blood sample. In this illustrative method, a blood sample may be acquired, and provided to a blood sample reservoir, as shown at step 300. Next, the blood sample may be provided to an anti-coagulant coated capillary 302 in a removable cartridge, and diluted. The blood sample may be provided to a white blood cell (WBC) measurement channel 340.

In the illustrative WBC measurement channel 340, the red blood cells may be lysed, and dye injected as appropriate, as shown at 342. The cells may then be hydrodynamically focused and provided single file down a WBC cytometry channel 344 in the removable cartridge. A light source 346, such as a vertical cavity surface emitting laser (VCSEL), may shine light on the individual cells as they pass by an analysis region of the WBC cytometry channel 344. In some cases, an array of VCSEL devices may be provided, and the VCSEL(s) that is/are aligned with the individual cells as they pass by the analysis region of the WBC cytometry channel 344 is/are activated.

As the individual cells/particles pass through the focused incident light beam, some of the light is blocked, scattered or otherwise obstructed, which can be detected by a detector (not shown). When two or more light sources are focused on different spaced spots along the WBC cytometry channel 344, the leading and/or trailing edge of each cell can be detected. By measuring the time it takes for a cell to traverse the distance from one focused spot to the next, the flow rate and thus the cell velocity can be determined. With the cell velocity determined, the length of time that a cell blocks, scatters or otherwise obstructs the light beam can be correlated to cell size and/or cell volume as shown at 348.

In some examples, a light source 350 and associated optics and/or polarizers may be provided. Light sources 346 and 350 may be combined into one light source (and even into one beam for the measurements desired) where all of the measurements may be done at the same time and on the same cell. The associated optics of light source 350 may collimate the light, and measure off-axis scatter, such as SALS, FALS and LALS scatter, as shown at 354. Like above, the SALS, FALS and LALS scatter may be used to measure, for example, the number of white blood cells counted (NWBC) 352, as well as to help with white blood cell differentiation, as shown at 356. In some cases, one or more polarizers is/are provided to polarize the light provided by the light source, and the level of polarization extinction/rotation detected at the detector may be used to help perform white blood cell differentiation, but this is not necessarily required in all examples. Also, fluorescent light from some of the cells (i.e., dyed, marked or tagged) may be detected as shown at 355.

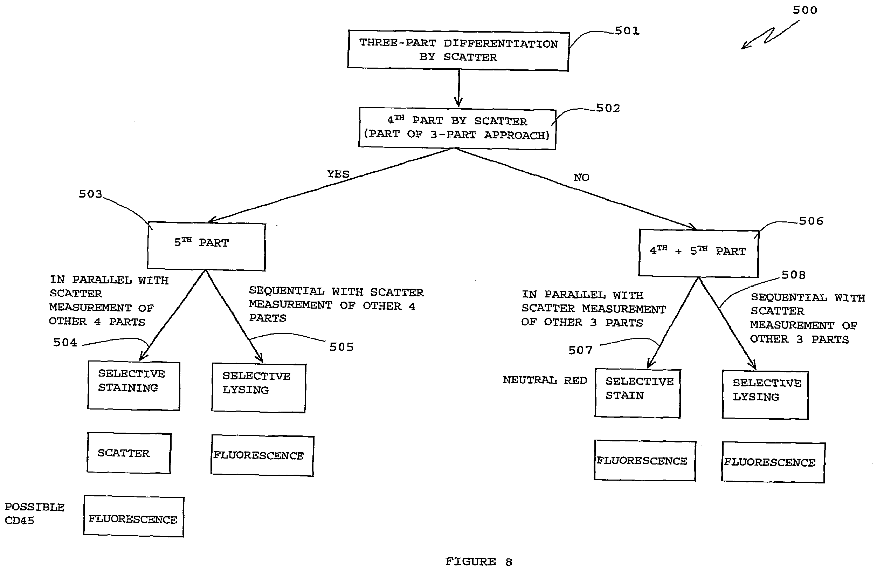

Figure 8 is an outline of a white blood cell five part differentiation measurement plan 500. To start, there may be a three part differentiation 501 by scatter. The fourth part differentiation 502 may be by scatter, such as along with the three part approach. So if the answer is "yes" to the fourth part by scatter, then one may go to the fifth part block 503. From block 503, the determination may be in the direction of arrow 504 where the fifth part determination is done in parallel with the scatter measurement for the other four parts. The parallel approach for the fifth part may include selective staining, CD45 with fluorescence, and scatter. The determination from block 503 may instead be in the direction of arrow 505 where the fifth part determination is done in sequence with the scatter measurement of the other four parts. Such fifth part measurement may be selective lysing or fluorescing.

If the answer to the block 502 question of whether the fourth part is by scatter, such as along with the three part scatter, is "no", then one may follow the arrow to block 506 indicating that the fourth and fifth part differentiation is yet to be done. In the direction of arrow 507, in parallel with scatter measurement of three parts, the differentiation for the fourth and fifth parts may be done with selective stain such as neutral red and/or effected with fluorescence which may involve a use of an appropriate stain. On the other hand, the fourth and fifth part differentiation may be done in a sequential fashion relative to the scatter measurement of the other three parts, in the direction of arrow 508. The sequential differentiation may be done with selective lysing and/or fluorescence.

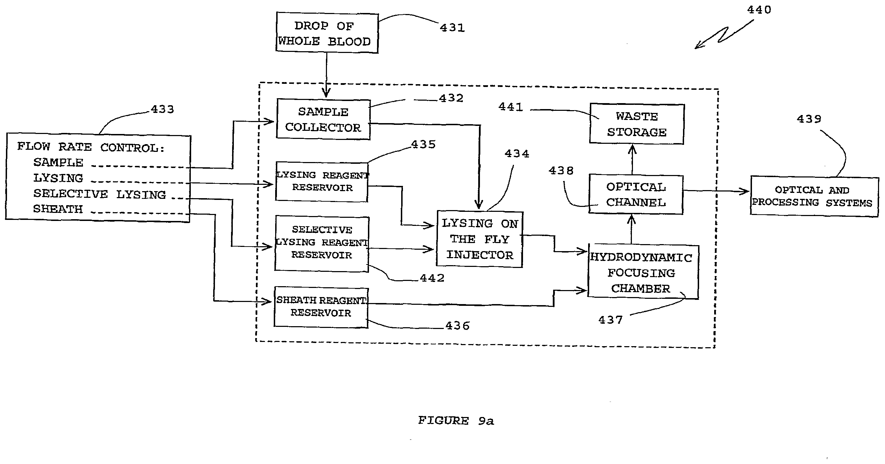

Figure 9a shows a version of the removable card or cartridge of a configuration 440 similar to the configuration 430 of Figure 4a. Configuration 440 may provide a four part differentiation of the cells flowing through the optical channel 438. It involves selective lysing of the sample from the collector 432. To provide this selective lysing is a selective lysing reagent reservoir 442 that may provide the selective lysing reagent to the lysing on the fly injector 434. The flow rate of the selective lysing reagent from the reservoir 442 may be controlled by the flow rate control mechanism 433. There may be two sequential runs on one sample of blood. The first run with the lysing may result in a three part differentiation. A second run on the same sample with selective lysing may provide a fourth part differentiation of the blood cells moving through the optical channel 438.

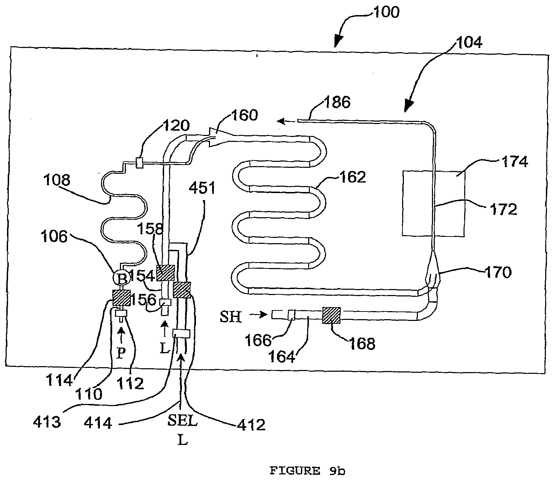

Figure 9b is similar to Figure 4b relative to their implementations of the configurations 440 and 430 in Figures 9a and 4a, respectively. The additional features of the implementation in Figure 9b may be noted. That is, the selective lysing reagent 414 may be provided to channel 451 which is connected to the channel 154 used for conveying the first lysing reagent to the intersecting region 160. A valve 413 on channel 451 may control when the selective lysing reagent 414 may flow to region 160. A flow sensor 412 may monitor the flow of the selective lysing reagent and provide a signal indicative of the flow to a flow control mechanism.

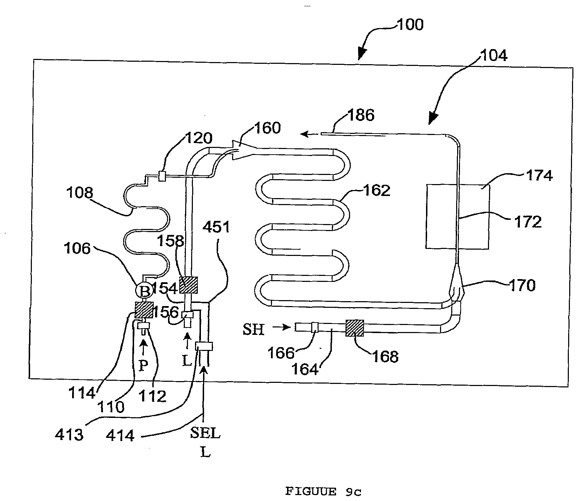

Figure 9c is similar to Figure 9b in that the flow sensor 412 is not present and the channel 451 for conveying the selective lysing reagent 414 is connected to the lysing reagent chamber tube 154 upstream of the flow sensor 158. Flow sensor 158 may be used for the lysing run and for the selective lysing run which may occur at different or separate times.

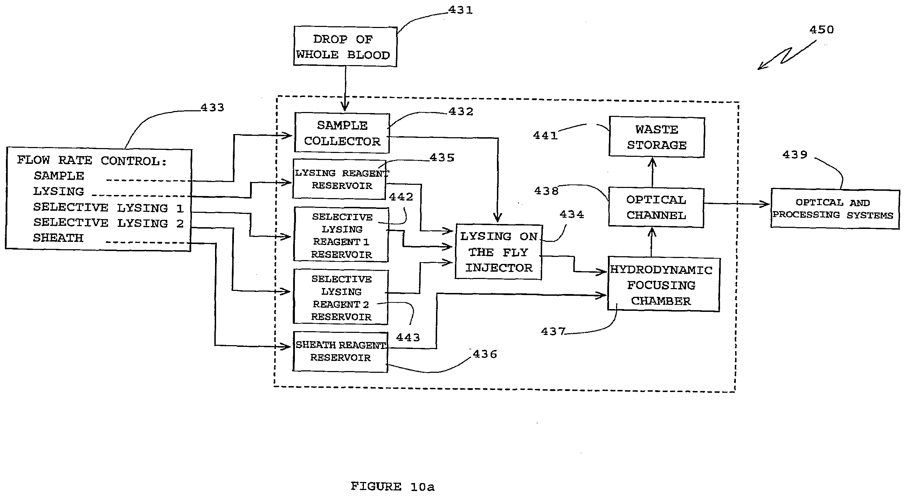

Figure 10a is a diagram or schematic of a configuration 450 which may provide a five part differentiation of white blood cells with three sequential runs on one sample. A first run with lysing from the lysing reagent reservoir 435 may result in a three part differentiation of the blood cells in the optical channel 438. A second run with a first selective lysing from the reagent reservoir 442 may result in a fourth part differentiation of the blood cells in channel 438. A third run with a second selective lysing from another selective lysing reagent reservoir 443 may result in a fifth part differentiation of the blood cells in the channel 438.

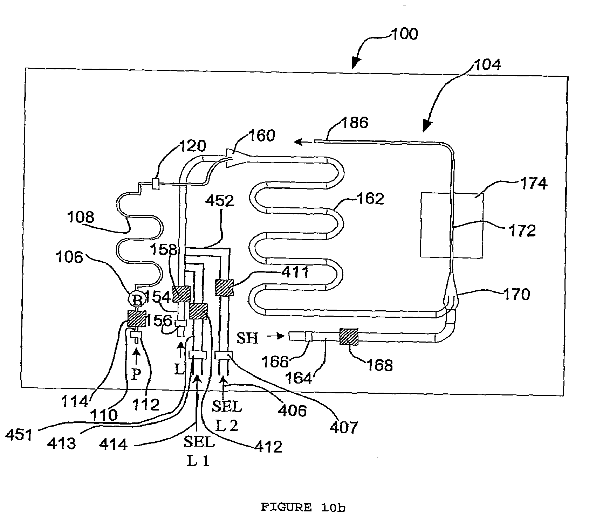

Figure 10b is similar to Figure 9b relative to their implementations of the configurations 450 and 440 in Figure 10a and 9a, respectively. The additional feature in Figure 10b is the second selective lysing agent 406 that is provided to the channel 452 which conveys the lysing reagent via channel 154 to the intersecting region 160. A valve 407 on channel 452 may control when the second selective lysing reagent 406 is to flow to region 160. A flow sensor 411 may monitor the flow of the second selective lysing reagent 406 through channel 452 and provide a signal indicative of the flow to a flow control mechanism.

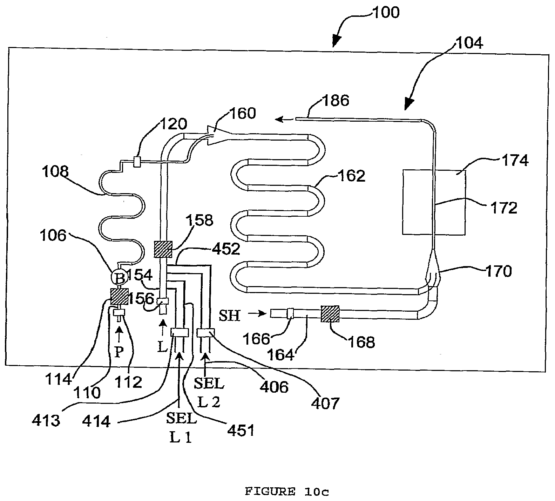

Figure 10c is similar to Figure 10b in that the flow sensors 412 and 411 are not present and the tubes or channels 451 and 452 for conveying the first selective lysing reagent 414 and the second selective lysing reagent 406, respectively, are connected to the lysing reagent channel 154 upstream from the flow sensor 158. Flow sensor 158 may be used for the lysing run, the first selective lysing run and the second selective lysing run which may occur at different times.

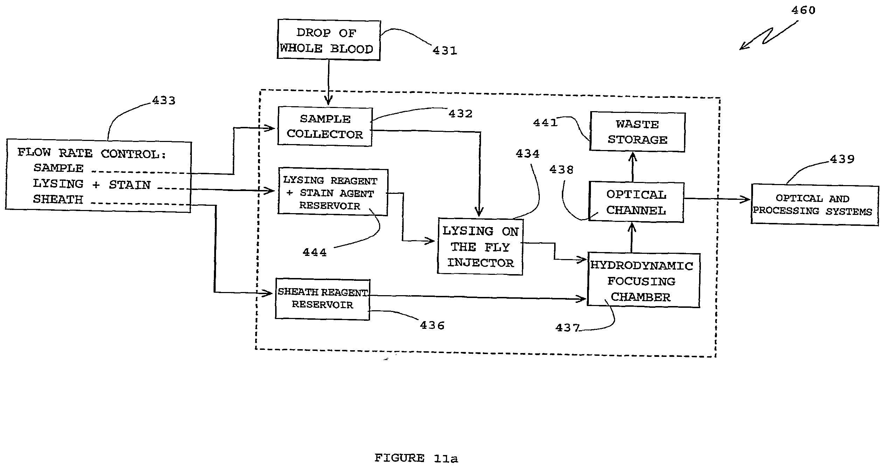

Figure 11a is a diagram or schematic of a configuration 460 which may provide a four part differentiation of blood cells with one run on a sample. There may be a reservoir 444 that contains a staining/fluorescence agent mixed in with the lysing reagent, which may be provided to the lysing on the fly injector 434. Except for this mixture of reservoir 444 and the absence of reservoir 435, the configuration 460 is similar to configuration 430 of Figure 4a. The three part scatter and the fourth part staining/fluorescence measurements may be done in parallel.

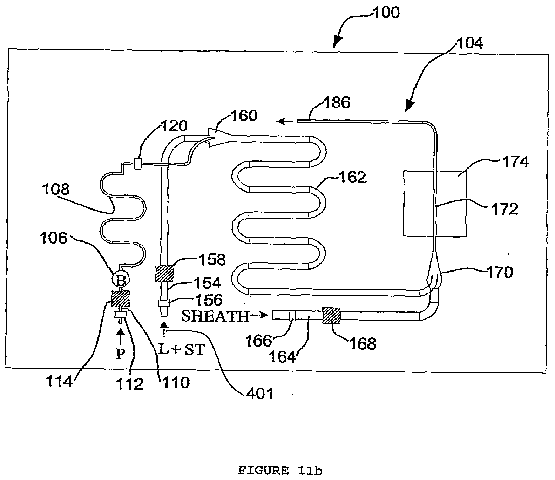

Figure 11b shows an implementation of the configuration 460 of Figure 11a. Figure 11b is similar to Figure 4b except that, rather than the lysing reagent, there is instead a mixture 401 of the lysing reagent and stain agent input to the channel 154.

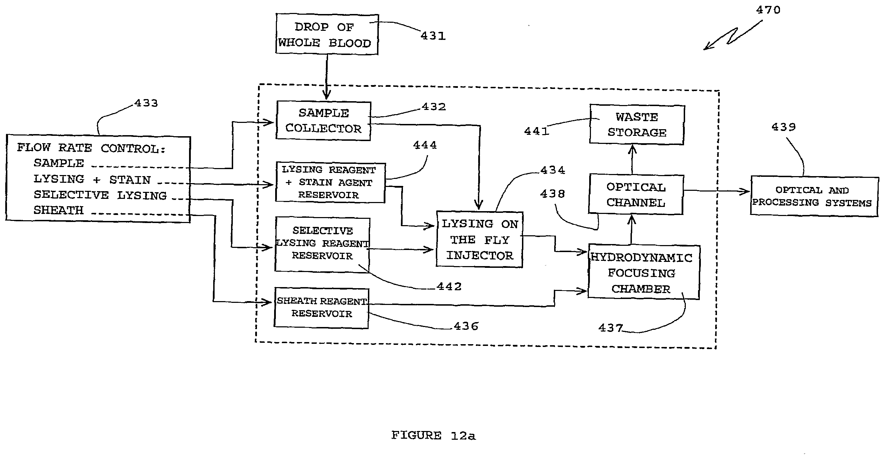

Figure 12a is a diagram or schematic of a configuration 470 which may provide a five part differentiation of blood cells with two sequential runs on one sample. The lysing reagent and stain agent mixture reservoir 444 may be retained from Figure 11a. A selective lysing reagent reservoir 442 is added (as in Figure 9a). Both reservoirs 442 and 444 may provide the selective lysing reagent and the mixture of lysing reagent and stain agent which may be provided via respective tubes or channels, but not necessarily in the same run, to the lysing on the fly injector 434. A first run with the mixture of the stain agent and the lysing reagent may provide the fourth part differentiation with fluorescence/scatter detection in addition to the three part differentiation with scatter detection of the blood cells in optical channel 438. A second run with the selective lysing reagent may provide a fifth part differentiation of the cells.

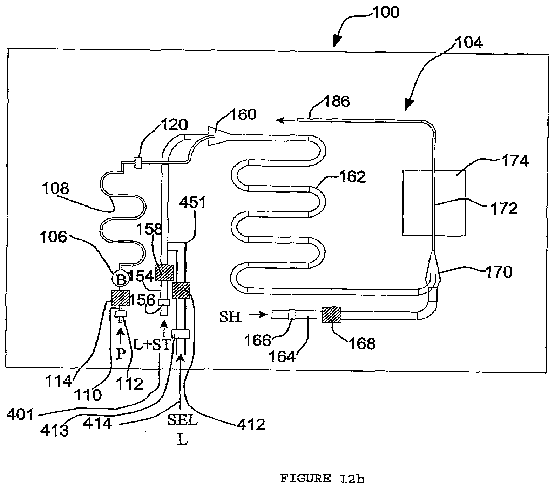

Figure 12b shows an implementation of the configuration 470 of Figure 12a. Figure 12b may be similar to Figure 11b in that it has a mixture 401 of lysing reagent and stain agent to channel 154 via valve 156. There may also be a flow sensor 158 for monitoring the flow of the mixture 401 with signals to a flow rate control mechanism. There may also be a selective lysing reagent 414 to a channel 451 via a valve 413 similar to that of Figure 9b. The reagent 414 may go through a flow sensor 412 via the channel 451 to the channel 154. Sensor 412 may monitor the flow of reagent 414 with signals to the flow rate control mechanism.

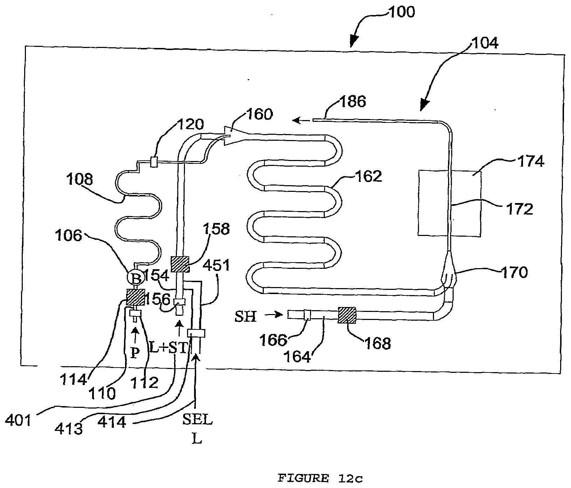

Figure 12c may be similar to the implementation in Figure 12b except that flow sensor 412 for monitoring the selective lysing reagent 414 is removed. Also, the channel 451 for conveying the reagent is coupled to channel 154 upstream from the flow sensor 158 for the mixture 401 of the lysing reagent and stain agent. The same flow sensor can be used for both the stain mixed with the lysing reagent 401 and the selective lysing reagent 414 since they may occur as separate runs at different times.

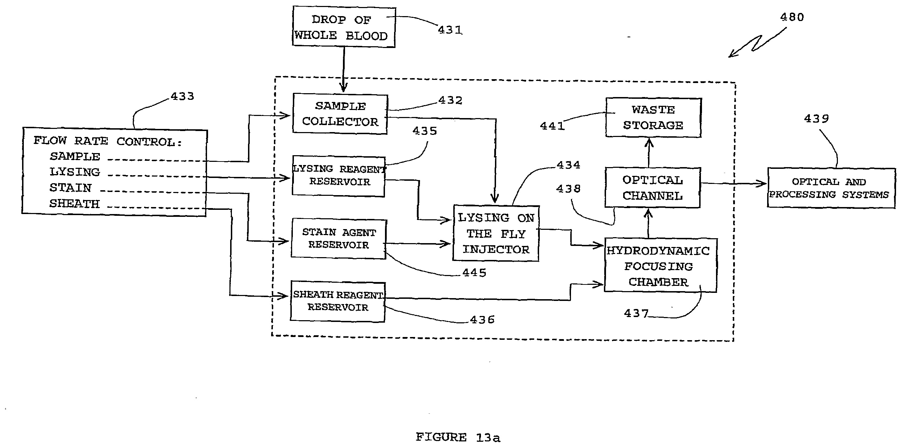

Figure 13a is a diagram or schematic of a configuration 480 which may provide a four part differentiation of cells in the optical channel 438. This configuration 480 may be similar to configuration 430 of Figure 4a except that configuration 480 additionally has a stain agent reservoir 445 with an output to the lysing on the fly injector 434. Also, the reservoir 445 is connected to the flow rate control mechanism 433 for controlling the flow of the stain agent to the injector mechanism 434. Configuration 480 may have a first run with a lysing reagent from reservoir 435 for a three part differentiation of the blood cells in the optical channel 438. A second run utilizing a staining of the blood cells with a stain agent from reservoir 445 and fluorescence/scatter observation of the cells in the optical channel 438 may provide a fourth part differentiation of the cells.

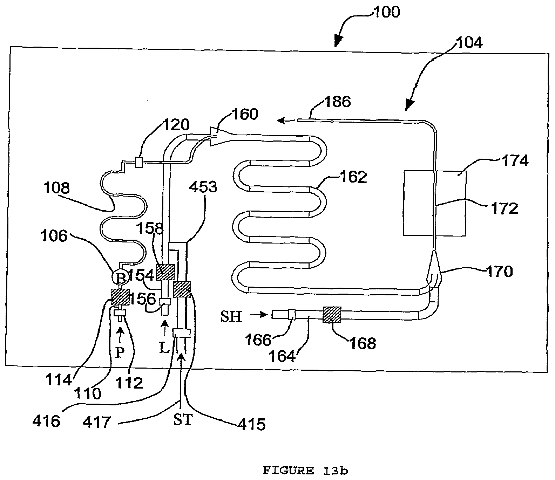

Figure 13b shows an implementation of the configuration 480 of Figure 13a. From a structural perspective, the layout of card 100 appears similar to that of Figure 9b except that a stain agent 417 is input via a channel 453 into channel 154 rather than a selective lysing reagent 414. Staining agent 417 may enter the channel 453 via a valve 416 and a flow sensor 415 onto the tube 154 and intersecting region 160.

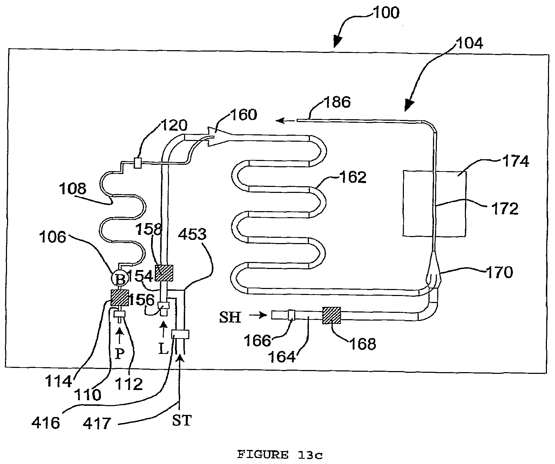

Figure 13c may be similar to Figure 13b except that the flow sensor 415 for monitoring the stain agent flow is absent and the channel 453 for conveying the stain agent 417 is connected to the channel 154 upstream from the flow sensor 158. The lysing reagent and stain agent may utilize the same flow sensor 158 since the lysing reagent and the agent 417 may be used in two separate runs at different times.

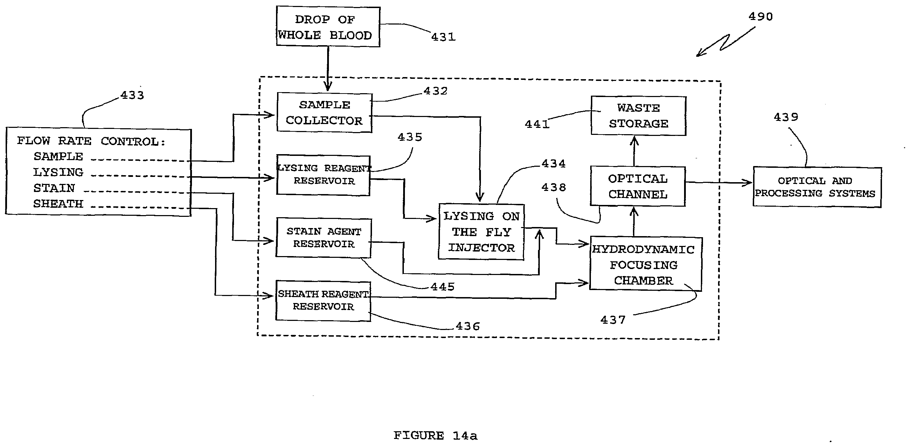

Figure 14a is a diagram or schematic of a configuration 490 which may provide a four part differentiation of the blood cells in the optical channel 438. Configuration 490 may be similar to configuration 480 except that the output of the strain agent reservoir 445 is not connected to the input of the lysing on the fly injector mechanism 434 but rather it is connected to a channel between the injector mechanism 434 output and an input of the hyrodynamic focusing chamber 437.

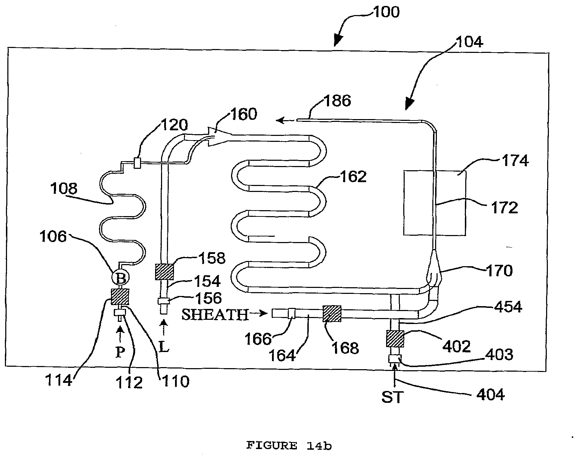

Figure 14b shows an implementation of the configuration 390 of Figure 14a. The implementation in Figure 14b may be similar to the implementation in Figure 4b except for a channel 454 connected to a channel 162 at a place just upstream from the intersecting region 170. This channel 454 may be connected to a stain agent reservoir and convey stain agent 404 to channel 162, via a valve 403 and flow sensor 402. Sensor 402 may provide signals about agent 404 flow to the flow rate control mechanism.

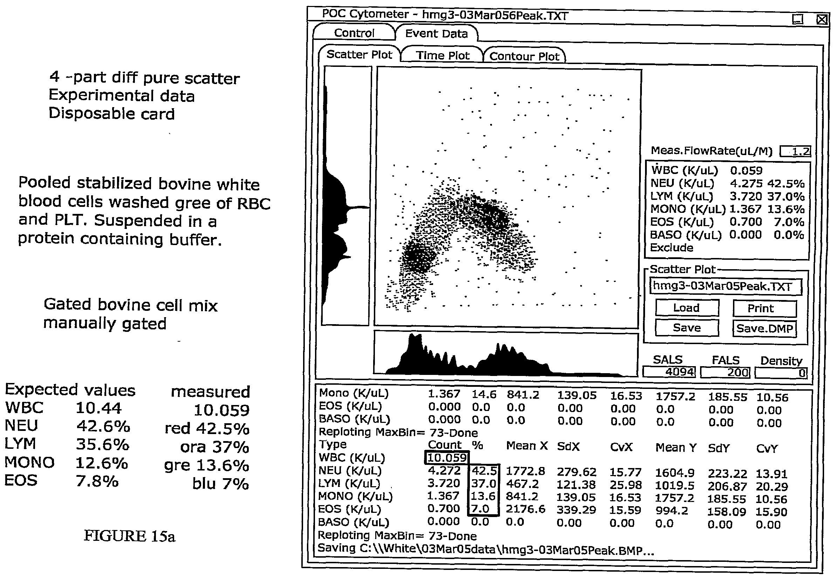

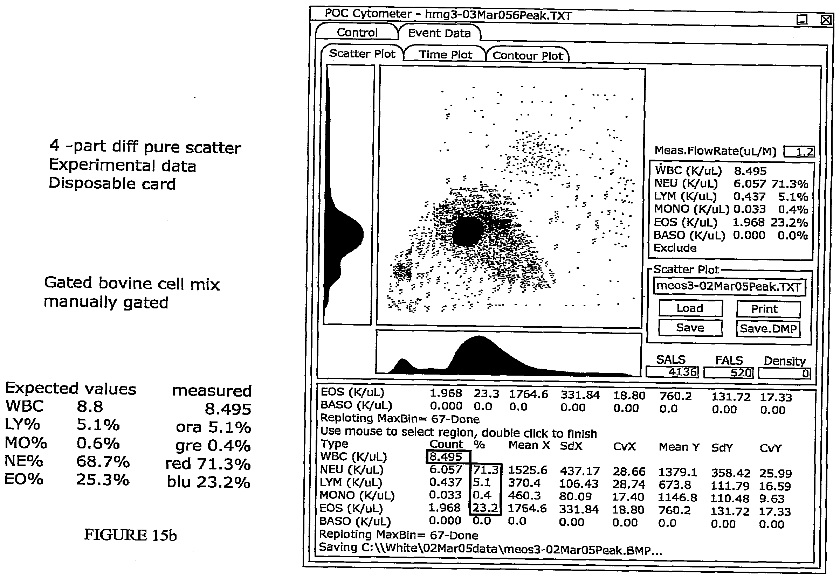

Figures 15a and 15b reveal data and plots of four-part differentiation of blood cells, utilizing configurations similar to those discussed herein.

It may be noted that the configurations of Figures 4a, 4b, 9a, 9b, 9c, 10a, 10b, 10c, 11a, 11b, 12a, 12b, 12c, 13a, 13b, 13c, 14a and 14b, may be representative of various approaches used for multipart differentiation of blood cells. Other configurations, incorporating a variety of permutations of the configurations disclosed herein, and other arrangements may be used to for multipart differentiation of blood cells.

In the present specification, some of the matter may be of a hypothetical or prophetic nature although stated in another manner or tense,

Although the invention has been described with respect to at least one illustrative example, many variations and modifications will become apparent to those skilled in the art upon reading the present specification. It is therefore the intention that the appended claims be interpreted as broadly as possible in view of the prior art to include all such variations and modifications.

## Claims

1. A fluid analysis apparatus (10) comprising:
a disposable card (14,100);
an optical fluidic channel (172, 438) situated in the card (14,100);
a first coupling region (160, 434) situated in the card (14,100);
a fluid sample source (108, 431) connected to the first coupling region (160, 434);
a lysing reagent source (64, 435) connected to the first coupling region (160, 434);
a lysing channel (154) connected to the first coupling region (160, 434);
a sheath reagent source (68, 436);
a second coupling region (170, 437) connected to the lysing channel (154) and to the sheath reagent source (68, 436); the optical fluidic channel (172, 438) connected to the second coupling region (170, 437); and
a light source (22a, 22b, 22c, 236, 346, 350) and detector (24a, 24b, 24c, 238, 239) (439) proximate to the optical fluidic channel (172, 438), wherein light scatter measurements from the detector (24a, 24b, 24c, 238, 239) provide three part differentiation (lymphocytes, monocytes and neutrophils) of white blood cells **characterized in that** the apparatus further comprises a selective lysing reagent source (64, 435) connected to the first coupling region (160, 434), wherein the light scatter measurements provide a fourth part (eosinophil) differentiation of the white blood cells, the apparatus further comprising a second selective lysing reagent source (64, 435) connected to the first coupling region (160, 434), wherein the light scatter measurements provide a fifth part (basophil) differentiation of the white blood cells, and wherein light measurements from the detector (24a, 24b, 24c, 238, 239) are possible from all angles including ninety degrees.

2. The apparatus (10) of claim 1, further comprising a stain agent source (66, 445) connected to the first coupling region (160, 434).

3. The apparatus (10) of claim 2, wherein the detector (24a, 24b, 24c, 238, 239) provides light absorption or light fluorescence measurements for a fourth part (eosinophil) and fifth part (basophil) differentiation of the white blood cells.

4. A method for measurement in a disposable card (14,100), comprising:
providing a blood sample (62, 233, 300, 431);
lysing red blood cells (342) of the blood sample (62, 233, 300, 431);
focusing white blood cells into a single file;
providing light (346, 350) to the white blood cells; and
obtaining parameters of the white blood cells from light scattered by the white blood cells using the apparatus (10) of any of claims 1, 2 or 3.

5. The method of claim 4, further comprising:
staining the blood (62, 233, 300, 431); and
obtaining additional parameters from light absorption or light fluorescence from stain on the white blood cells; and
wherein the additional parameters comprise a fourth part (eosinophil) differentiation of the white blood cells and/or a fifth part (basophil) differentiation of the white blood cells.

6. The method of claim 4, further comprising:
staining the white blood cells;
obtaining additional parameters from light fluorescence or light absorption from stain on the white blood cells; and
wherein the additional parameters comprise a fourth part (eosinophil) differentiation of the white blood cells and/or a fifth part (basophil) differentiation of the white blood cells.

## Patentansprüche

1. Flüssigkeitsanalysevorrichtung (10), umfassend:
eine Einwegkarte (14, 100);
einen optischen Flüssigkeitskanal (172; 438), der sich in der Karte (14,100) befindet;
einen ersten Verbindungsbereich (160, 434), der sich in der Karte (14, 100) befindet;
eine Flüssigkeitsprobenquelle (108, 431), die mit dem ersten Verbindungsbereich (160, 434) verbunden ist;
eine Lysierungsreagensquelle (64, 435), die mit dem ersten Verbindungsbereich (160, 434) verbunden ist;
einen Lysierungskanal (154), der mit dem ersten Verbindungsbereich (160, 434) verbunden ist;
eine Hüllreagensquelle (68, 436);
einen zweiten Verbindungsbereich (170, 437), der mit dem Lysierungskanal (154) und mit der Hüllreagensquelle (68, 436) verbunden ist;
den optischen Flüssigkeitskanal (172, 438), der mit dem zweiten Verbindungsbereich (170, 437) verbunden ist; und
eine Lichtquelle (22a, 22b, 22c, 236, 346, 350) und einen Detektor (24a, 24b, 24c, 238, 239) (439) nahe dem optischen Flüssigkeitskanal (172, 438), wobei Lichtstreuungsmessungen vom Detektor (24a, 24b, 24c, 238, 239) eine Differenzierung von drei Teilen (Lymphozyten, Monozyten und Neutrophile) weißer Blutzellen bereitstellen,
**dadurch gekennzeichnet, dass** die Vorrichtung ferner eine mit dem ersten Verbindungsbereich (160, 434) verbundene selektive Lysierungsreagensquelle (64, 435) bereitstellt, wobei die Lichtstreuungsmessungen eine Differenzierung eines vierten Teils (Eosinophile) der weißen Blutzellen bereitstellen,
wobei die Vorrichtung ferner eine mit dem ersten Verbindungsbereich (160, 434) verbundene zweite selektive Lysierungsreagensquelle (64, 435) bereitstellt, wobei die Lichtstreuungsmessungen eine Differenzierung eines fünften Teils (Basophile) der weißen Blutzellen bereitstellen und wobei die Lichtmessungen vom Detektor (24a, 24b, 24c, 238, 239) aus allen Winkeln einschließlich neunzig Grad möglich ist.

2. Vorrichtung (10) nach Anspruch 1, ferner umfassend eine Anfärbungsmittelquelle (66, 445), die mit dem ersten Verbindungsbereich (160, 434) verbunden ist.

3. Vorrichtung (10) nach Anspruch 2, wobei der Detektor (24a, 24b, 24c, 238, 239) Lichtabsorptions- oder Lichtfluoreszenzmessungen für eine Differenzierung des vierten Teils (Eosinophile) und fünften Teils (Basophile) der weißen Blutzellen bereitstellt.

4. Verfahren für eine Messung in einer Einwegkarte (14,100), umfassend:
Bereitstellen einer Blutprobe (62, 233, 300, 431),
Lysieren roter Blutzellen (342) der Blutprobe (62, 233, 300, 431),
Bündeln weißer Blutzellen in eine einzige Reihe,
Bereitstellen von Licht (346; 350) an die weißen Blutzellen; und
Gewinnen von Parametern der weißen Blutzellen aus Licht, das von den weißen Blutzellen mithilfe der Vorrichtung (10) nach einem der Ansprüche 1, 2 oder 3 gestreut wird.

5. Verfahren nach Anspruch 4, ferner umfassend:
Anfärben des Blutes (62, 233, 300, 431); und
Gewinnen zusätzlicher Parameter aus einer Lichtabsorption oder Lichtfluoreszenz aus der Anfärbung der weißen Blutzellen; und
wobei die zusätzlichen Parameter eine Differenzierung eines vierten Teils (Eosinophile) der weißen Blutzellen und/oder eine Differenzierung eines fünften Teils (Basophile) der weißen Blutzellen umfassen.

6. Verfahren nach Anspruch 4, ferner umfassend:
Anfärben der weißen Blutzellen;
Gewinnen zusätzlicher Parameter aus der Lichtfluoreszenz oder Lichtabsorption aus der Anfärbung der weißen Blutzellen; und
wobei die zusätzlichen Parameter eine Differenzierung eines vierten Teils (Eosinophile) der weißen Blutzellen und/oder eine Differenzierung eines fünften Teils (Basophile) der weißen Blutzellen umfassen.

## Revendications

1. Appareil d'analyse de fluide (10) comprenant :
une carte jetable (14, 100);
un canal fluidique optique (172, 438) situé dans la carte (14, 100) ;
une première région d'accouplement (160, 434) située dans la carte (14, 100);
une source d'échantillon de fluide (108, 431) reliée à la première région d'accouplement (160, 434);
une source de réactif de lyse (64, 435) reliée à la première région d'accouplement (160, 434);
un canal de lyse (154) relié à la première région d'accouplement (160, 434);
une source de réactif de gaine (68, 436);
une seconde région d'accouplement (170, 437) reliée au canal de lyse (154) et à la source de réactif de gaine (68, 436);
le canal fluidique optique (172, 438) étant connecté à la seconde région d'accouplement (170, 437); et
une source de lumière (22a, 22b, 22c, 236, 346, 350) et un détecteur (24a, 24b, 24c, 238, 239) (439) à proximité du canal fluidique optique (172, 438),
des mesures de diffusion de lumière provenant du détecteur (24a, 24b, 24c, 238, 239) fournissant une différenciation de trois parties (lymphocytes, monocytes et neutrophiles) de globules blancs
**caractérisé en ce que** l'appareil comprend en outre
une source de réactif de lyse sélective (64, 435) reliée à la première région d'accouplement (160, 434), les mesures de diffusion de lumière fournissant une différenciation de quatrième partie (éosinophile) des globules blancs,
l'appareil comprenant en outre une seconde source de réactif de lyse sélective (64, 435) reliée à la première région d'accouplement (160, 434), les mesures de diffusion de lumière fournissant une différenciation de cinquième partie (basophile) des globules blancs, et les mesures de lumière provenant du détecteur (24a, 24b, 24c, 238, 239) étant possibles depuis tous les angles y compris à quatre-vingt-dix degrés.

2. Appareil (10) selon la revendication 1, comprenant en outre une source d'agent de coloration (66, 445) reliée à la première région d'accouplement (160, 434).

3. Appareil (10) selon la revendication 2, dans lequel le détecteur (24a, 24b, 24c, 238, 239) fournit des mesures d'absorption de lumière ou de fluorescence de lumière pour une différenciation de quatrième partie (éosinophile) et de cinquième partie (basophile) des globules blancs.

4. Procédé de mesure dans une carte jetable (14, 100), consistant à :
fournir un échantillon de sang (62, 233, 300, 431);
lyser des globules rouges (342) de l'échantillon de sang (62, 233, 300, 431);
centrer les globules blancs dans une seule file;
fournir de la lumière (346, 350) sur les globules blancs; et
obtenir des paramètres des globules blancs à partir de la lumière diffusée par les globules blancs à l'aide de l'appareil (10) selon l'une quelconque des revendications 1, 2 ou 3.

5. Procédé selon la revendication 4, consistant en outre à :
colorer le sang (62, 233, 300, 431); et
obtenir des paramètres supplémentaires de l'absorption de lumière ou de la fluorescence de lumière provenant de la coloration sur les globules blancs; et
dans lequel les paramètres supplémentaires comprennent une différenciation de quatrième partie (éosinophile) des globules blancs et/ou une différenciation de cinquième partie (basophile) des globules blancs.

6. Procédé selon la revendication 4, consistant en outre à :
colorer les globules blancs;
obtenir des paramètres supplémentaires de la fluorescence de lumière ou de l'absorption de lumière à partir de la coloration sur les globules blancs; et
dans lequel les paramètres supplémentaires comprennent une différenciation de quatrième partie (éosinophile) des globules blancs et/ou une différenciation de cinquième partie (basophile) des globules blancs.
